# EUROPEAN PATENT APPLICATION

(11) **EP 4 169 582 A1**
(43) Date of publication of application: **26.04.2023**
(21) Application number: 22201357.5
(22) Date of filing: 13.10.2022
(51) Int. Cl.: A62B 9/00, A62B 18/00, A62B 18/04, A62B 18/08, A61B 5/11

(54) **PROTECTIVE EQUIPMENT FOR RESPIRATION AND MANAGEMENT METHOD**

(30) Priority: 22.10.2021 JP 2021172894
(71) Applicant: Yamamoto Kogaku Co., Ltd., Osaka 577-0056 (JP)
(72) Inventor: HASHIMOTO, Hiroki, Osaka, 5770056 (JP); SAKAMOTO, Kenichi, Osaka, 5770056 (JP)
(74) Representative: SSM Sandmair

(57) **Abstract**

An object of the present invention is to provide protective equipment for respiration, a protective system for respiration, and a management method that can assist equipment management for stably supplying filtered air to an operator or safety management for securing safety of work performed while receiving the supply of the filtered air such that the equipment management or the safety management can be appropriately performed even from a remote place. Protective equipment for respiration 1 of the present application includes a protective equipment main body 10 that covers the face of an operator, a fan unit 20 including an air filter 21 and an electric fan 22, a detecting section 30, a first control section 40, and a first communication section 50. The detecting section 30 detects a physical quantity concerning at least one of a work environment of the operator, an action state of the operator, and an operation state of the electric fan 22. The first control section 40 controls the electric fan 22 based on a detection result of the detecting section 30. The first communication section 50 transmits first information indicating a detection result of the detecting section 30 or second information concerning an operation amount on a control target of the first control section 40 to an external device.

## Description

### BACKGROUND OF THE INVENTION

### Field of the Invention

The present invention relates to protective equipment for respiration that supplies filtered air filtered by an air filter to an operator, a protective system for respiration that performs equipment management for such protective equipment for respiration or safety management for work in which such protective equipment for respiration is used, and a management method.

### Description of the Related Art

As protective equipment for respiration that supplies filtered air filtered by an air filter to an operator working under an environment with a lot of dust such as a welding site, Japanese Patent Laid-Open No. 2021-119889 (Patent Literature 1) describes protective equipment for respiration including a protective equipment main body and an electric fan. The protective equipment main body covers the head and the face of the operator. The electric fan sends the filtered air to the protective equipment main body via a hose. With the protective equipment for respiration described in Patent Literature 1, it is possible to supply the filtered air filtered by the air filter to the operator and protect the operator under the environment with a lot of dust such as the welding site.

However, in the protective equipment for respiration described in Patent Literature 1, equipment management for replacing a clogged air filter in an appropriate period and sufficiently charging a battery or replacing an old battery such that the electric fan can blows the air without stopping until a work end is necessary. The fact is that, when there are many pieces of equipment that should be managed, the equipment management described above takes a long time and the replacement of the air filter and the battery is performed according to determination of an individual operator. For example, operators visually determine clogging of the air filter. The replacement of the air filter is not performed according to a uniform standard.

Further, in a work site with high temperature such as a welding site, safety management for, for example, optimizing a work schedule to prevent accidents from occurring because of excessive fatigue of operators and checking whether the operators are unable to act because of heatstroke or the like is necessary. However, it is conceivable that an administrator involved in the safety management described above often engages in another job in a place apart from the work site and cannot quickly take measures in a crisis of an operator.

The present invention has been devised in view of the problems described above, and an object of the present invention is to provide protective equipment for respiration, a protective system for respiration, and a management method that can assist equipment management for stably supplying filtered air to an operator or safety management for securing safety of work performed while receiving the supply of the filtered air such that the equipment management or the safety management can be appropriately performed even from a remote place.

### SUMMARY OF THE INVENTION

Protective equipment for respiration disclosed in the present application includes a protective equipment main body, a fan unit, a detecting section, a first control section, and a first communication section. The protective equipment main body covers a face of an operator. The fan unit includes an air filter and an electric fan. The air filter filters air. The electric fan blows the filtered air to the protective equipment main body via ventilation means such as a hose. The filtered air is the air filtered by the air filter. The detecting section is provided in the fan unit and detects a physical quantity concerning at least one of a work environment of the operator, an action state of the operator, and an operation state of the electric fan. The first control section controls the electric fan based on a detection result of the detecting section. The first communication section transmits first information indicating a detection result of the detecting section or second information concerning an operation amount on a control target of the first control section to an external device including information output means.

In the protective equipment for respiration disclosed in the present application, the detecting section includes an air volume sensor. The air volume sensor detects a supplied air volume of the filtered air blown to the protective equipment main body via the ventilation means. The first control section adjusts, based on a detection result of the air volume sensor, electric energy supplied to the electric fan such that the supplied air volume of the filtered air reaches a predetermined reference air volume. The first communication section transmits the second information indicating an adjustment amount of the electric energy adjusted by the first control section to the external device as replacement period related information. The replacement period related information is information related to a replacement period of the air filter.

The protective equipment for respiration disclosed in the present application further includes a power supply section. The power supply section includes at least one battery and supplies electric power to the electric fan. In the protective equipment for respiration disclosed in the present application, the detecting section includes a voltage sensor. The voltage sensor detects a terminal voltage of the at least one battery. The first communication section transmits the first information indicating the terminal voltage detected by the voltage sensor to the external device as residual power related information. The residual power related information is information related to residual power of the at least one battery.

The protective equipment for respiration disclosed in the present application, the detecting section includes an acceleration sensor. The acceleration sensor detects acceleration about the fan unit. The first communication section transmits the first information indicating the acceleration detected by the acceleration sensor to the external device as action disability determination information. The action disability determination information is information for determining whether the operator has fallen down and has become disabled to act.

In the protective equipment for respiration disclosed in the present application, the detecting section includes a temperature sensor. The temperature sensor detects an air temperature in the work environment of the operator. The first communication section transmits the first information indicating the air temperature detected by the temperature sensor to the external device as heat stress index related information. The heat stress index related information is information related to calculation of a heat stress index.

A protective system for respiration disclosed in the present application includes the protective equipment for respiration described above and a management device functioning as the external device. The management device includes a second communication section, an information output section functioning as the information output means, and a second control section. The second communication section receives the first information or the second information transmitted by the first communication section. The information output section outputs third information concerning at least one of a work environment of the operator, an action state of the operator, and an operation state of the electric fan as visual information or auditory information. The second control section controls, based on the first information or the second information received by the second communication section, the information output section to output the third information.

In the protective system for respiration disclosed in the present application, the second communication section receives the second information indicating the adjustment amount of the electric energy as the replacement period related information. When the adjustment amount indicated by the second information is equal to or larger than a predetermined threshold for determining clogging of the air filter, the second control section controls the information output section to output replacement attention information as the third information. The predetermined threshold is a threshold for determining the clogging of the air filter. The replacement attention information is information for urging replacement of the air filter.

In the protective system for respiration disclosed in the present application, the second communication section receives the first information indicating the acceleration as action disability determination information. The action disability determination information is information for determining whether the operator has fallen down and has become disabled to act. When a change in the acceleration indicated by the first information matches a predetermined action disability determination pattern, the second control section controls the information output section to output action disability warning information as the third information. The action disability determination pattern is a pattern indicating that the operator has fallen down and has become disabled to act. The action disability warning information is information for warning that the operator has fallen down and has become disabled to act.

A management method disclosed in the present application is a management method for, using the protective equipment for respiration disclosed in the present application, performing equipment management for stably supplying filtered air to an operator or safety management for securing safety of work performed while receiving the supply of the filtered air, the management method including a first step, a second step, and a third step. The first step is a step of receiving the first information or the second information transmitted by the first communication section. The second step is a step of the information output means outputting third information concerning at least one of a work environment of the operator, an action state of the operator, and an operation state of the electric fan as visual information or auditory information. The third step is a step of controlling, based on the first information or the second information received in the first step, the information output means to output the third information.

With the protective equipment for respiration, the protective system for respiration, and the management method of the present invention, it is possible to assist equipment management for stably supplying filtered air to an operator or safety management for securing safety of work performed while receiving the supply of the filtered air such that the equipment management or the safety management can be appropriately performed even from a remote place.

### BRIEF DESCRIPTION OF THE DRAWINGS

FIG. 1 is a functional block diagram showing a protective system for respiration including protective equipment for respiration according to an embodiment of the present invention;
FIG. 2 is a perspective view showing the protective equipment for respiration according to the embodiment of the present invention;
FIG. 3 is an exploded perspective view showing a fan unit of the protective equipment for respiration according to the embodiment of the present invention;
FIG. 4 is a functional block diagram showing a detecting section of the protective equipment for respiration according to the embodiment of the present invention;
FIG. 5 is a graph showing a relation among a degree of clogging of an air filter, rotating speed of an electric fan, and electric energy supplied to the electric fan;
FIG. 6 is a graph showing a relation among the degree of clogging of the air filter, a blown air volume of the electric fan, and a reference air volume;
FIG. 7 is a conceptual diagram showing an output example in an information output section according to the embodiment;
FIG. 8 is a graph showing a detection result of an acceleration sensor about an action disability determination pattern for determining whether an operator has fallen down and has become disabled to act;
FIG. 9 is a flowchart showing main processing in the protective system for respiration according to the embodiment of the present invention; and
FIG. 10 is a flowchart showing fan control processing in the protective system for respiration according to the embodiment of the present invention.

### DETAILED DESCRIPTION OF THE PREFERRED EMBODIMENT

Protective equipment for respiration according to an embodiment of the present invention is explained in detail below with reference to the drawings. Note that the embodiment explained below is technically variously limited because the embodiment is a specific example preferred to carry out the present invention. However, the present invention is not limited to the embodiment unless it is clearly described in the following explanation in particular that the invention is limited.

### <Embodiment>

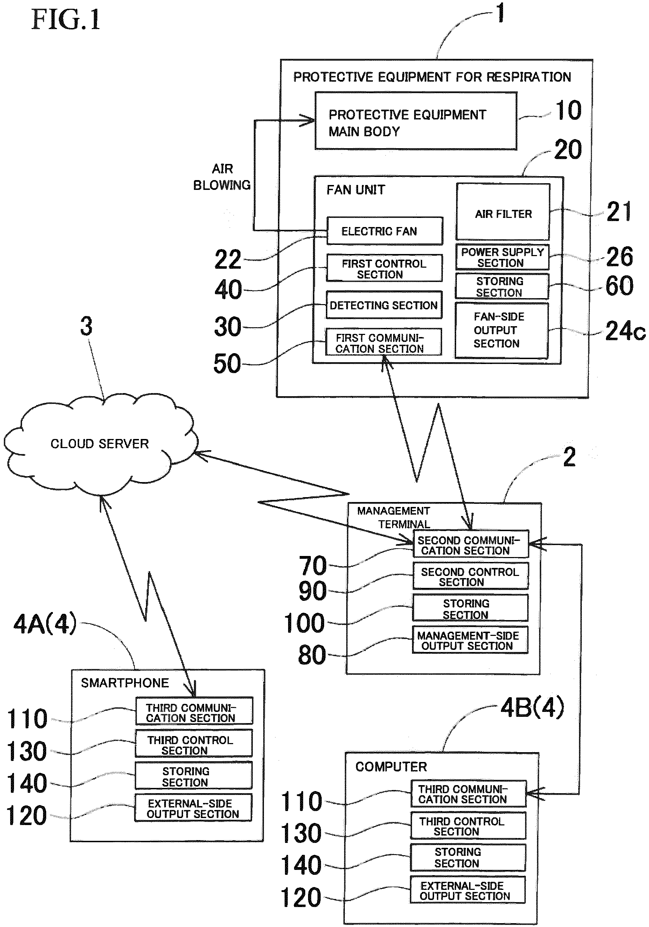

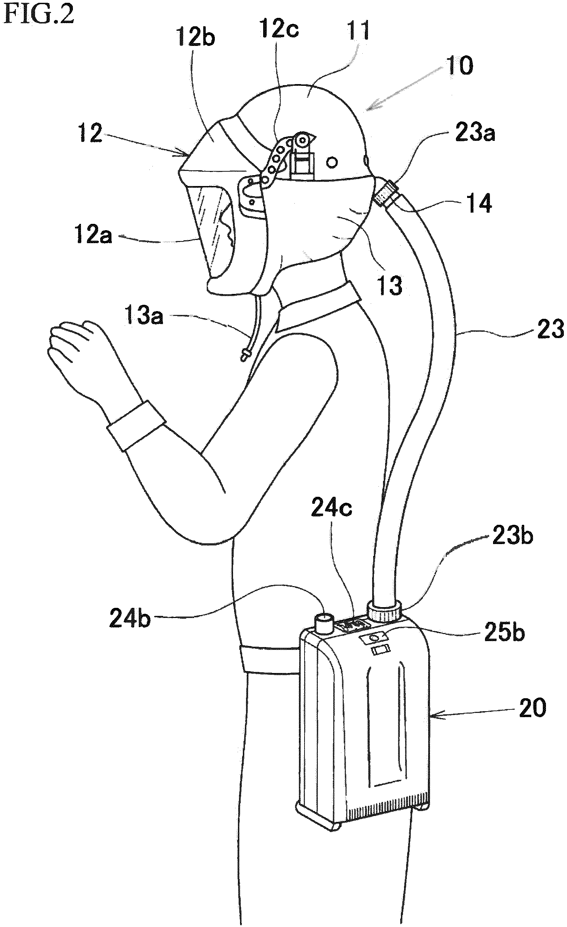

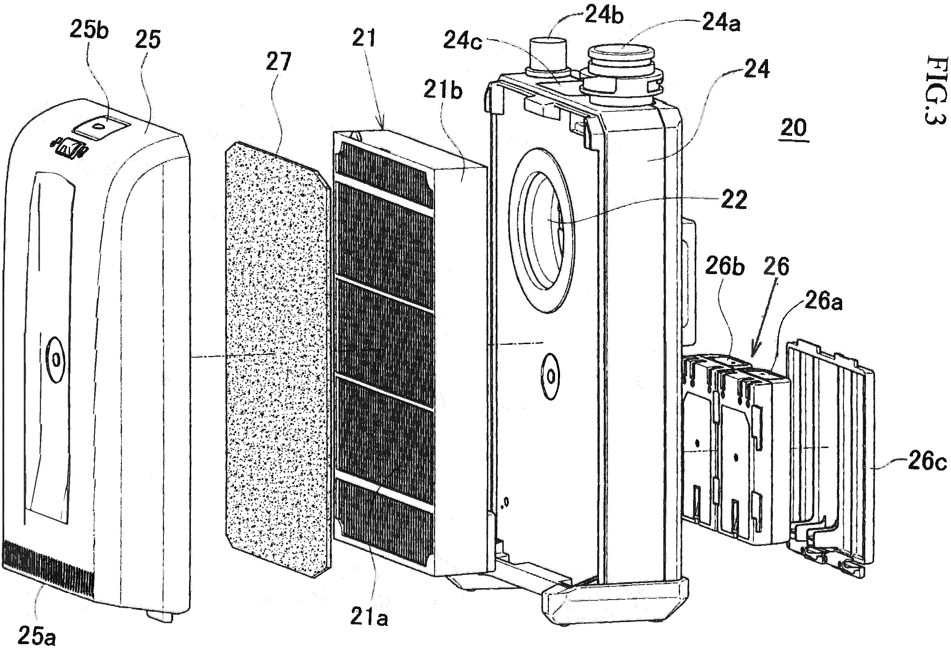

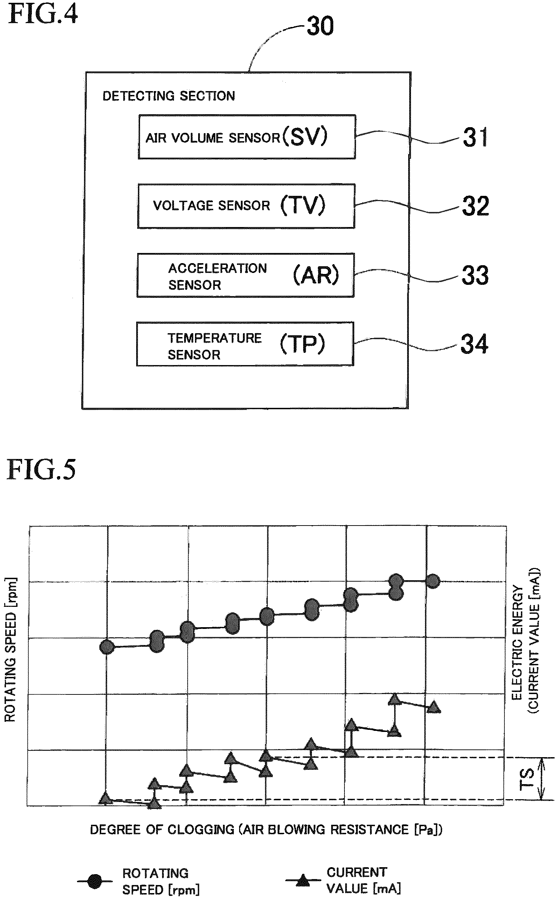

Protective equipment for respiration, a protective system for respiration, and a management method according to an embodiment of the present invention are explained below with reference to FIGS. 1 to 4. FIG. 1 is a functional block diagram showing the protective system for respiration including the protective equipment for respiration according to the embodiment. FIG. 2 is a perspective view showing the protective equipment for respiration according to the embodiment. FIG. 3 is an exploded perspective view showing a fan unit of the protective equipment for respiration according to the embodiment. FIG. 4 is a functional block diagram showing a detecting section of the protective equipment for respiration according to the embodiment.

As shown in FIGS. 1 and 2, the protective system for respiration according to the embodiment includes protective equipment for respiration 1, a management terminal 2 functioning as a management device, an external storage device such as a cloud server 3, and external terminals 4 such as a smartphone 4A and a personal computer 4B. Each of the external terminals 4 only has to be a computer and may be a tablet computer or a notebook personal computer.

The protective equipment for respiration 1 supplies filtered air to an operator under an environment with a lot of dust such as a welding factory or a welding site and protects the operator. The protective equipment for respiration 1 includes a protective equipment main body 10, a fan unit 20, a detecting section 30, a first control section 40, a first communication section 50, and a first storing section 60.

The protective equipment main body 10 covers the face of the operator. Note that, in the embodiment, as shown in FIG. 2, the protective equipment main body 10 includes a helmet 11, a face shield 12, a sealing hood 13, and an air receiving port 14. The protective equipment main body 10 covers the face to house, on the inside, the entire head including the face and the head of a wearing person. The air receiving port 14 is provided in the helmet 11.

As shown in FIG. 2, the face shield 12 includes a lens 12a and a frame body 12b. The lens 12a is hermetically attached to the frame body 12b. The frame body 12b is supported on the helmet 11 to be capable of turning up and down by a rotating hinge 12c and supports the lens 12a.

The helmet 11 and the frame body 12b are hermetically in contact. Filtered air is introduced into, via the air receiving port 14, an internal space of the protective equipment main body 10 surrounded by the helmet 11, the face shield 12, and the sealing hood 13. The filtered air is supplied to the operator. The filtered air is air filtered by an air filter 21 explained below. A not-shown opening section through which the neck of the wearing person is inserted is present in the sealing hood 13. A gap between the opening section of the sealing hood 13 and the neck can be reduced by tightening an adjustable strap 13a.

As shown in FIG. 3, the fan unit 20 includes an air filter 21 and an electric fan 22. The fan unit 20 includes a unit main body 24, a filter cover 25, a power supply section 26, and a prefilter 27.

The air filter 21 filters air. In the embodiment, the air filter 21 includes a filter main body 21a formed from nonwoven fabric and a filter frame 21b that supports the filter main body 21a. In the embodiment, the filter frame 21b is formed from resin and integrally molded with the filter main body 21a.

The electric fan 22 blows filtered air to the protective equipment main body 10 via ventilation means such as a hose 23. In the embodiment, adjustment of the rotating speed of the electric fan 22 is performed to adjust an electric current to the electric fan 22 with a pulse width modulation scheme and adjusting electric energy EQ supplied to the electric fan 22.

The hose 23 includes, as shown in FIGS. 2 and 3, an air receiving-side coupling section 23a and an air blowing-side coupling section 23b at both end portions. The air receiving-side coupling section 23a is coupled to the air receiving port 14. The air blowing-side coupling section 23b is coupled to an air blowing port 24a of the fan unit 20.

As shown in FIG. 3, the unit main body 24 incorporates the electric fan 22 and the power supply section 26 and includes the air blowing port 24a described above, an adjustment knob 24b, and a fan-side output section 24c. The adjustment knob 24b receives operation for turning on and off the electric fan 22 and operation for adjusting a blown air volume (a supplied air volume SV). The fan-side output section 24c outputs, as visual information or auditory information, various kinds of information such as a blown air volume of the electric fan 22, presence or absence of clogging of the air filter 21, residual power of a battery or the like in the power supply section 26, a communication state of the first communication section 50, and information about whether various alarms explained below are emitted. The fan-side output section 24c can be formed from an indicator, a liquid crystal panel, a speaker, or the like.

The filter cover 25 includes an air intake port 25a and holds the air filter 21 in an air supply path from the intake port 25a to the electric fan 22. The filter cover 25 is detachably attachable to the unit main body 24. It is possible to detach the filter cover 25 from the unit main body 24 by operating a detachment button 25b.

The power supply section 26 includes at least one battery and supplies electric power to sections of the fan unit 20 including the electric fan 22. In the embodiment, the power supply section 26 includes two batteries, that is, a first battery 26a and a second battery 26b, and is held on the inside of the unit main body 24 by a battery lid 26c. In the embodiment, the first battery 26a and the second battery 26b supply electric power to the electric fan 22 and the like with a switching scheme in such a manner that one of the first battery 26a and the second battery 26b having a lower terminal voltage TV supplies electric power to the electric fan 22 and the like and, when the terminal voltage TV drops to a predetermined voltage, the other starts supply of electric power.

The prefilter 27 is placed in an air supply path from the intake port 25a to the air filter 21 and protects the air filter 21 from, for example, sputtering in a welding site.

The detecting section 30 is provided in the fan unit 20 and detects a physical quantity concerning at least one of a work environment of the operator, an action state of the operator, and an operation state of the electric fan 22.

As shown in FIG. 4, in the embodiment, the detecting section 30 includes an air volume sensor 31, a voltage sensor 32, a six-axis acceleration sensor 33, and a temperature sensor 34. Note that the acceleration sensor 33 may be a three-axis sensor. The detecting section 30 may further include a humidity sensor and an optical sensor.

The air volume sensor 31 detects the supplied air volume SV of filtered air blown to the protective equipment main body 10 via the ventilation means such as the hose 23. The voltage sensor 32 detects the terminal voltage TV of the first battery 26a and the second battery 26b. The acceleration sensor 33 detects acceleration AR about the fan unit 20 that moves according to movement of the operator. The temperature sensor 34 detects an air temperature TP in a work environment of the operator.

The first control section 40 controls the electric fan 22 based on a detection result of the detecting section 30. In the embodiment, the first control section 40 is formed from a microcomputer and adjusts, based on a detection result of the air volume sensor 31, for example, with a pulse width modulation scheme, the electric energy EQ supplied to the electric fan 22 from the power supply section 26 such that the supplied air volume SV of the filtered air reaches a predetermined reference air volume RV. In this embodiment, the reference air volume RV is set by the first control section 40 according to operation on the adjustment knob 24b.

The first communication section 50 transmits first information indicating a detection result of the detecting section 30 or second information concerning an operation amount on a control target of the first control section 40 to the management terminal 2 functioning as the management device, which is the external device, in the claims. A communication scheme for transmitting the first information or the second information from the first communication section 50 to the management terminal 2 may be wired communication in which a wired LAN or the like is used or may be wireless communication in which a wireless LAN, a wireless telephone line, or the like is used. However, for workability improvement, at least the first communication section 50 preferably has a wireless communication function such as Wi-Fi.

In the embodiment, the first communication section 50 transmits the first information indicating the supplied air volume SV detected by the air volume sensor 31 to the management terminal 2 and transmits the second information indicating an adjustment amount DA of the electric energy EQ, which is an operation amount on the control target of the first control section 40, to the management terminal 2 as replacement period related information RI1. The replacement period related information RI1 is information related to a replacement period of the air filter 21.

The first communication section 50 transmits the first information indicating the terminal voltage TV detected by the voltage sensor 32 to the management terminal 2 as residual power related information RI2. The residual power related information RI2 is information related to residual power of the first battery 26a and the second battery 26b or a charging state ([%]).

Further, the first communication section 50 transmits the first information indicating the acceleration AR detected by the acceleration sensor 33 to the management terminal 2 as action disability determination information RI3. The action disability determination information RI3 is information for determining whether the operator has fallen down and has become disabled to act.

Further, the first communication section 50 transmits the first information indicating the air temperature TP detected by the temperature sensor 34 to the management terminal 2 as heat stress index related information RI4. The heat stress index related information RI4 is information related to calculation of a heat stress index.

The first storing section 60 stores various programs such as a driving control program for the electric fan 22 and a discharge control program in the power supply section 26 and stores various data such as an initial duty ratio (rotating speed or a current value) corresponding to the reference air volumes RV designated by operation on the adjustment knob 24b, increase values in stages at the time when the duty ratio is increased stepwise as explained below, and initialization data used in initialization processing performed when the fan unit 20 is turned on. The programs and the data stored in the first storing section 60 are read out at any time by the first control section 40 according to necessity.

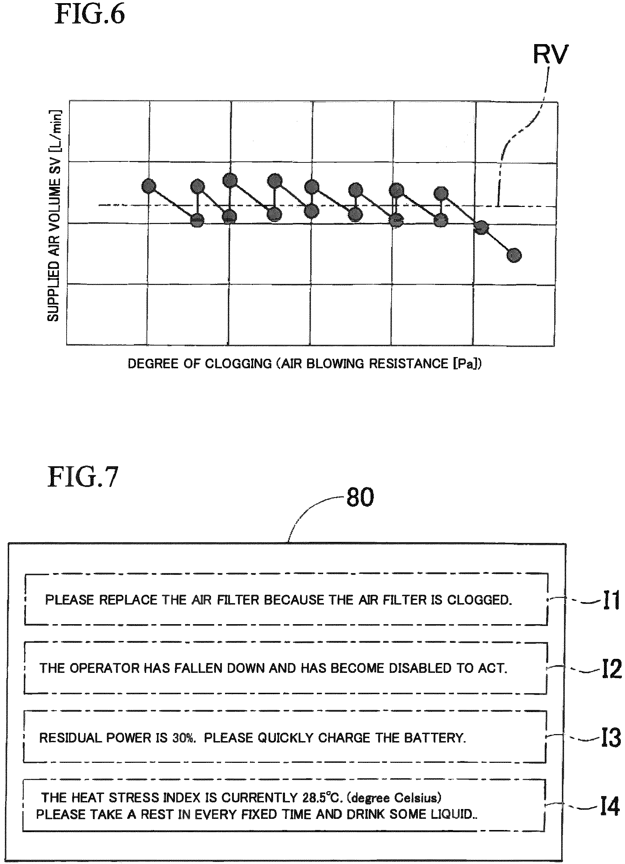

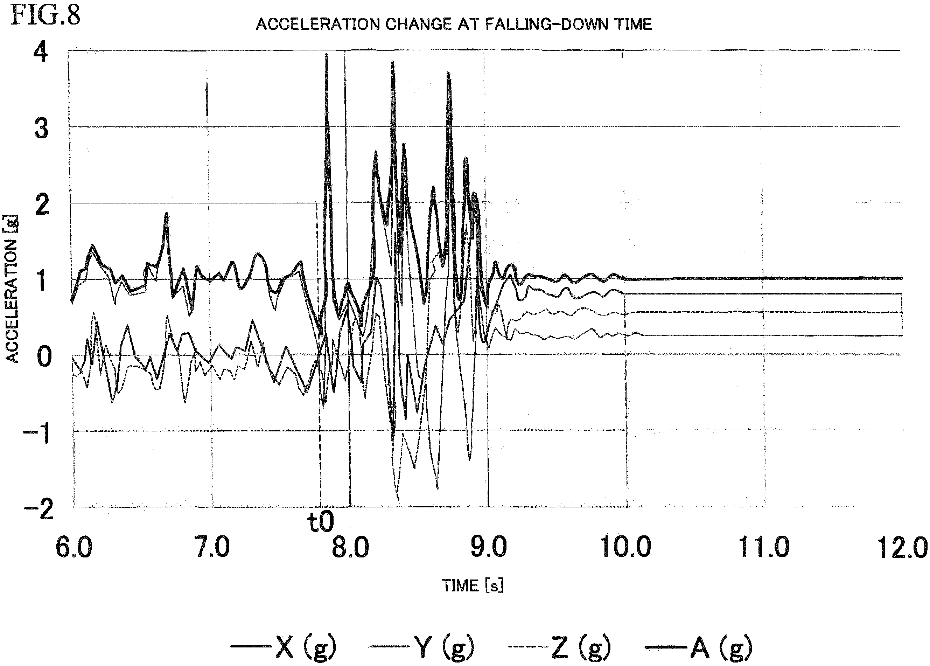

Subsequently, the management terminal 2, the external terminal 4, and the management method according to the embodiment are explained with reference to FIGS. 5 to 8 together with FIGS. 1 to 4 explained above. FIG. 5 is a graph showing a relation among a degree of clogging of an air filter, rotating speed of an electric fan, and electric energy supplied to the electric fan. FIG. 6 is a graph showing a relation among the degree of clogging of the air filter, a blown air volume (a supplied air volume) of the electric fan, and a reference air volume. FIG. 7 is a conceptual diagram showing an output example in an information output section according to the embodiment. FIG. 8 is a graph showing a detection result of an acceleration sensor about an action disability determination pattern for determining whether an operator has fallen down and has become disabled to act.

As shown in FIG. 1, the management terminal 2 includes a second communication section 70, a management-side output section 80 functioning as an information output section, which is information output means, a second control section 90, and a second storing section 100. In the embodiment, the management terminal 2 is realized by installing, in a general-purpose terminal such as a smartphone, a tablet computer, a personal computer, or a notebook personal computer, an application or software including programs for causing a computer to function as sections explained below. The same applies to the external terminal 4. Therefore, the protective system for respiration and the management method of the present invention are sometimes realized by the application or the software.

When the protective system for respiration and the management method of the present invention are realized by the application or the software, the programs configuring the application or the software may be recorded in a removable medium such as a CD-ROM or a USB memory and distributed to a user or may be distributed by being downloaded to a computer of the user such as the management terminal 2 and the external terminal 4 via a network. Further, these programs may be provided to, without being downloaded, the computer of the user as a web service provided via the network.

In the present specification and the like, "section", "means", "device", and "system" do not simply mean physical means but also include a case in which functions of the "section", the "means", the "device", and the "system" are realized by software. Functions of one "section", one "means", one "device", and one "system" may be realized by two or more physical means or devices or functions of two or more "sections", two or more "means", two or more "devices", and two or more "systems" may be realized by one physical means or one device.

The second communication section 70 receives the first information or the second information transmitted by the first communication section 50. A communication scheme for the second communication section to receive the first information or the second information from the first communication section 50 may be wired communication in which a wired LAN or the like is used or may be wireless communication in which a wireless LAN, a wireless telephone line, or the like are used. However, in particular, when the management terminal 2 is a portable terminal such as a smartphone or a notebook personal computer, for convenience of the administrator, the second communication section 70 preferably has a wireless communication function such as Wi-Fi.

In the embodiment, the second communication section 70 receives the first information indicating the supplied air volume SV detected by the air volume sensor 31 and receives, as the replacement period related information RI1, the second information indicating the adjustment amount DA of the electric energy EQ. The second communication section 70 receives, as the residual power related information RI2, the first information indicating the terminal voltage TV detected by the voltage sensor 32. Further, the second communication section 70 receives, as the action disability determination information RI3, the first information indicating the acceleration AR detected by the acceleration sensor 33. Further, the second communication section 70 receives, as the heat stress index related information RI4, the first information indicating the air temperature TP detected by the temperature sensor 34.

In the embodiment, the second communication section 70 transmits the first information, the second information, and the third information to the external storage device such as the cloud server 3. The external storage device such as the cloud server 3 stores the first information, the second information, and the third information transmitted by the second communication section 70.

The management-side output section 80 outputs, as visual information or auditory information, the third information concerning at least one of the work environment of the operator, the work state of the operator, and the operation state of the electric fan 22. The management-side output section 80 can be formed from a monitor, a liquid crystal screen, a speaker, or the like included in the management terminal 2.

The second control section 90 controls the management-side output section 80 to output the third information based on the first information or the second information received by the second communication section 70. The control performed by the first control section 40 and the second control section 90 is explained below.

As shown in FIG. 5, air blowing resistance [Pa] of the electric fan 22 increases as a degree of clogging of the air filter 21 increases. Therefore, in order to keep the supplied air volume SV at the reference air volume RV, the rotating speed [rpm] of the electric fan 22 needs to be increased and the electric energy EQ (for example, a current value [mA]) supplied to the electric fan 22 needs to be increased as the degree of the clogging of the air filter 21 increases.

As a result, as shown in FIG. 6, when the first control section 40 is performing control for adjusting the electric energy EQ supplied to the electric fan 22 to keep the supplied air volume SV at the reference air volume RV, the degree of the clogging of the air filter 21 excessively increases and, if the electric energy EQ supplied to the electric fan 22 to keep the supplied air volume SV at the reference air volume RV cannot be further increased, the supplied air volume SV greatly decreases.

Therefore, as shown in FIG. 5, when the adjustment amount DA for the electric energy EQ is equal to or larger than a predetermined threshold TS, as shown in FIG. 7, the second control section 90 controls the management-side output section 80 to output replacement attention information I1 serving as the third information. Note that the threshold TS is a threshold for determining clogging of the air filter 21.

As shown in FIG. 7, the replacement attention information I1 is information for urging replacement of the air filter 21 and may be information for urging replacement of the air filter 21 as visual information with a sentence such as "Please replace the air filter because the air filter is clogged.", flashing of a screen, or the like, may be information for communicating the sentence described above with voice as auditory information, or may be information for urging replacement of the air filter 21 with predetermined alarm sound or the like.

As shown in FIG. 7, when a change in the acceleration AR indicated by the first information matches a predetermined action disability determination pattern JP, the second control section 90 controls the management-side output section 80 to output action disability warning information 12 serving as the third information. The predetermined action disability determination pattern JP is a pattern indicating that the operator has fallen down and has become disabled to act.

The action disability warning information 12 is information for warning that the operator has fallen down and has become disabled to act and may be information for communicating, with a sentence such as "The operator has fallen down and has become disabled to act.", flashing of a screen, or the like, that the operator has fallen down and has become disabled to act as visual information, may be information for communicating the sentence described above with voice as auditory information, or may be information for communicating, with predetermined alarm sound or the like, that the operator has fallen down and has become disabled to act.

FIG. 8 shows a change in the acceleration AR at the time when the operator actually wears the protective equipment for respiration 1, falls down, and stands still. The action disability determination pattern JP has a condition "a", a condition "b", and a condition "c" described below. The change in the acceleration AR satisfies the condition "a", the condition "b", and the condition "c", whereby the change in the acceleration AR matches the action disability determination pattern JP and it is determined that the operator has fallen down and has become disabled to act.

As shown in FIG. 8, the action disability determination pattern JP includes three conditions, that is, the condition "a": at a certain point in time t0, combined acceleration A(g) of acceleration X(g) in the front-rear direction (the left-right direction in FIG. 3), acceleration Y(g) in the up-down direction, and acceleration Z(g) in the left-right direction of the fan unit 20 is equal to or smaller than a first determination reference value FJA [g] (g: gravitational acceleration), the condition "b": the combined acceleration A(g) reaches a second determination reference value IJA [g] or more within a first determination time IJT [s] from the point in time t0, and the condition "c": after a point in time when a second determination time TJT [s] elapses from the point in time t0, the acceleration Y(g) in the up-down direction is a third determination reference value TJA [g] or less and the combined acceleration A(g) is within a range of (1±0.05) g for a third determination time TTL [s].

The first determination reference value FJA [g] is a reference value for detecting a fall of an object and only has to be set to acceleration smaller than 1.0 g but is preferably set to acceleration of 0.8 g or less, more preferably set to acceleration of 0.5 g or more and 0.8 or less, and still more preferably set to acceleration of 0.7 g or more and 0.8 g or less in order to prevent wrong detection. The first determination time IJT [s] is a time for guaranteeing a causal relation between a fall of an object and collision with the ground that subsequently occurs and only has to be set to a time of 0.8 second or more and 1.2 seconds or less but is preferably set to a time of 0.9 second or more and 1.1 seconds or less in order to prevent wrong determination.

The second determination reference value IJA [g] is a reference value for detecting collision of a fallen object with the ground and only has to be set to acceleration of 2.5 g or more and 4.5 g or less but is preferably set to acceleration of 3.0 g or more and 4.0 g or less in order to prevent wrong detection. The second determination time TJT [s] is a time for waiting for a shock due to the collision to stop and only has to be set to a time of 1.8 seconds or more and 2.2 seconds or less but is preferably set to a time of 1.9 seconds or more and 2.1 seconds or less in order to prevent wrong determination.

The third determination time TTL [s] is a time for determining whether an object stands still and only has to be set to a time of 1.8 seconds or more and 2.2 seconds or less but is preferably set to a time of 1.9 seconds or more and 2.1 seconds or less in order to prevent wrong determination. The third determination reference value TJA [g] is a reference value for detecting a roll of an object and only has to be set to acceleration of 0.40 g or more and 0.60 g or less but is preferably set to acceleration of 0.45 g or more and 0.55 g or less in order to prevent wrong detection.

In other words, the condition "a" indicates an accelerating motion in the gravity direction of the fan unit 20, the condition "b" indicates a shock to the fan unit 20, and the condition "c" indicates that the fan unit 20 is tilted a predetermined angle or more and stands still. Therefore, in the embodiment, when a detection result of the acceleration sensor 33 matches the action disability determination pattern JP indicating the accelerating motion in the gravity direction of the fan unit 20, subsequent occurrence of the shock to the fan unit 20, and a subsequent tilt at the predetermined angle or more and a subsequent standstill of the fan unit 20, the second control section 90 controls the management-side output section 80 to output the action disability warning information 12 as the third information.

In the embodiment, the second control section 90 controls the management-side output section 80 to output, based on the terminal voltage TV indicated by the first information, the residual power (the charging state [%]) of the first battery 26a and the second battery 26b as visual information or auditory information.

As shown in FIG. 7, when the terminal voltage TV of both of the first battery 26a and the second battery 26b indicated by the first information drops to the predetermine voltage or less, the second control section 90 controls the management-side output section 80 to output, as visual information or auditory information, charging attention information 13 serving as the third information. The charging attention information 13 is information, content of which is the residual power of the first battery 26a and the second battery 26b, or information, content of which is to urge charging of the first battery 26a and the second battery 26b.

When a capacity of the first battery 26a and the second battery 26b decreases from an initial capacity by a predetermined rate, the second control section 90 may control the management-side output section 80 to output, as visual information or auditory information, battery replacement attention information (for example, "Please quickly replace the battery.") related to replacement of the first battery 26a and the second battery 26b.

Further, the second control section 90 calculates a heat stress index concerning heatstroke based on information indicating the air temperature TP indicated by the first information and, as shown in FIG. 7, controls the management-side output section 80 to output, as visual information or auditory information, a work schedule and heatstroke warning information 14 related to warning of heatstroke serving as the third information. Note that, as a calculation formula of the heat stress index (WBGT), WBGT=0.735×TP+0.0374×RH+0.0029×TP×RH-4.064, where, RH: relative humidity, can be used.

Subsequently, the management method according to the embodiment of the present invention is explained. The management method in the embodiment is a management method for performing, using the protective equipment for respiration 1, equipment management for stably supplying filtered air to an operator or safety management for securing safety of work performed while receiving the supply of the filtered air. The management method includes a first step, a second step, and a third step.

The first step is a step of receiving first information or second information transmitted by the first communication section 50. The details of the first step are the same as the operation of the second communication section 70 explained above. The second step is a step of outputting third information concerning at least one of a work environment of the operator, an action state of the operator, and an operation state of an electric fan. The details of the second step are the same as the operation of the management-side output section 80 explained above. The third step is a step of controlling information output means to output the third information based on the first information or the second information received in the second step. The details of the third step are the same as the operation of the second control section 90 explained above.

Subsequently, the external terminal 4 such as the smartphone 4A, the personal computer 4B, or the like is explained with reference to FIG. 1 and the like.

The external terminal 4 includes a third communication section 110, an external-side output section 120, a third control section 130, and a third storing section 140. In the embodiment, like the management terminal 2, the external terminal 4 is realized by installing an application or software including programs for causing a computer to function as sections explained below. A method of providing the application or the software is the same as the method in the management terminal 2.

The third communication section 110 receives the first information, the second information, or the third information from the second communication section 70 of the management terminal 2 or the external storage device such as the cloud server 3. The external-side output section 120 includes a monitor device, a liquid crystal panel, and a speaker and outputs the first information, the second information, or the third information as visual information or auditory information. The third control section 130 controls the external-side output section 120 to output the first information, the second information, or the third information as visual information or auditory information. The third storing section 140 stores various programs and various data.

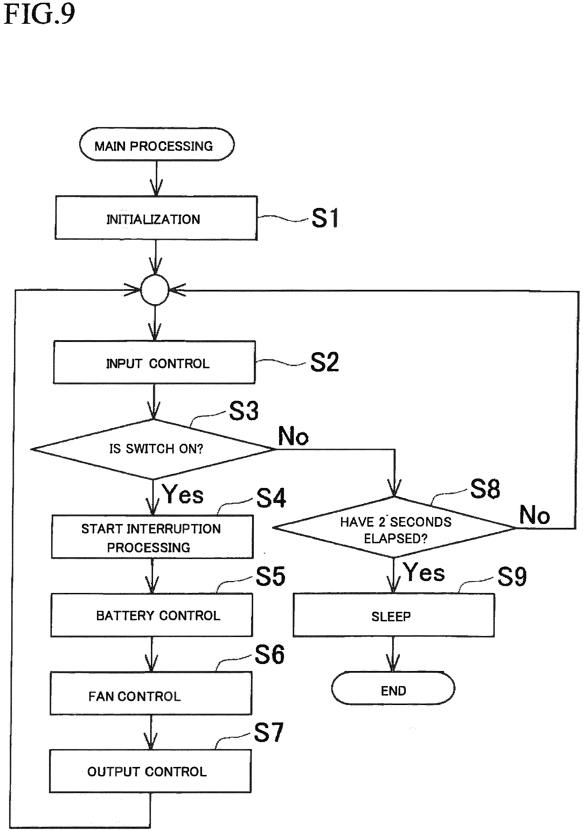

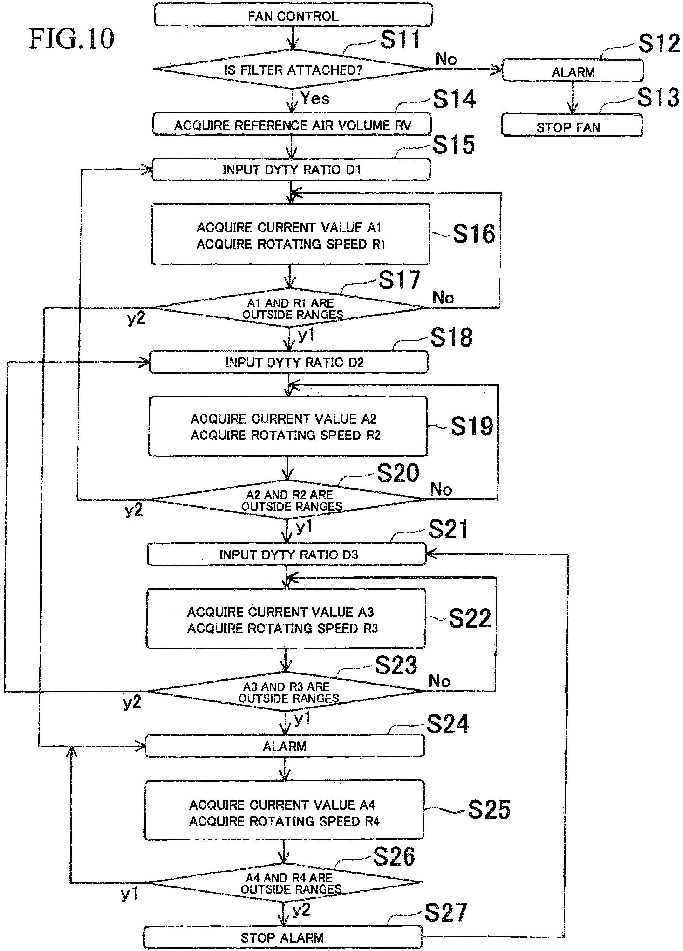

Subsequently, a flow of control in the embodiment is explained with reference to FIGS. 9 and 10 together with FIGS. 1 to 8. FIG. 9 is a flowchart showing main processing in the protective system for respiration according to the embodiment. FIG. 10 is a flowchart showing fan control processing in the protective system for respiration according to the embodiment.

In step S1 of the main processing shown in FIG. 9, a power switch of the fan unit 20 is turned on by the adjustment knob 24b and predetermined initialization processing such as reading of a control program and data for control from the first storing section 60 to the first control section 40 is performed.

In step S2 of the main processing shown in FIG. 9, predetermined input control processing is performed. In the input control processing in step S2, a blown air volume designated by the adjustment knob 24b is read and it is determined, according to, for example, a detection result of an optical sensor, whether the air filter 21 is correctly set. The blown air volume is switched stepwise in this embodiment to, for example, "weak", "medium", "strong", and "strongest".

In step S3 of the main processing shown in FIG. 9, it is determined whether the power switch of the fan unit 20 is on. If the power switch is on, the processing proceeds to step S4 and, if the power switch is not on, the processing proceeds to step S8.

In step S4 of the main processing in FIG. 9, various kinds of interruption processing such as interruption processing for controlling the rotating speed of the electric fan 22 with, for example, a pulse width modulation scheme are started.

In step S5 of the main processing shown in FIG. 9, predetermined battery control is executed. In the battery control in step S5, a battery having a lower terminal voltage TV of the two batteries, that is, the first battery 26a and the second battery 26b is selected and power supply is started.

In step S6 of the main processing shown in FIG. 9, predetermined fan control processing is executed. The fan control processing in step S6 is generally explained below with reference to FIG. 10.

As shown in FIG. 10, in the fan control processing in step S6, it is determined whether the air filter 21 is attached (step S11). When the air filter 21 is not attached ("No" in step S11), an alarm is emitted, the power supply to the electric fan 22 is stopped, and the electric fan 22 is stopped (step S13).

When the air filter 21 is attached (Yes in step S11), the reference air volume RV, which is a setting value of the blown air volume input in the input control in step S2, is read (step S14). An initial value D1 of the electric energy EQ (a duty ratio (DYTY ratio)) supplied to the electric fan 22 according to the reference air volume RV is determined (step S15). Operation of the electric fan 22 is started at a predetermined initial current value A1 and predetermined initial rotating speed R1 (step S16). The initial current value A1, the initial rotating speed R1, and set current values A2, A3, and A4 and set rotating speeds R2, R3, and R4 described below are stored in the first storing section 60 in advance as a rotating speed/current value table for each of blown air volumes (for example, "weak", "medium", "strong", and "strongest") switched by the adjustment knob 24b.

After the operation of the electric fan 22 is started, when actual rotating speed is larger than the initial rotating speed R1 by a predetermined amount or more and an actual current value is smaller than the initial current value A1 by a predetermined amount or more, it is determined that the supplied air volume SV does not reach the reference air volume RV ("y1" in step S17). The electric energy EQ (the duty ratio) supplied to the electric fan is increased stepwise (step S18 and step S21).

When an actual supplied air volume SV does not reach the reference air volume RV even if the electric energy EQ is increased stepwise a determined number of times ("y1" in step S20 and in step S23), assuming that the adjustment amount DA is equal to or larger than the threshold TS, the second control section 90 controls the management-side output section 80 to output the replacement attention information I1, which is an alarm about replacement of the air filter 21, (step S24) and continues the operation at a slightly larger current value while subsequently keeping a state in which the replacement attention information I1 is output unless the clogging decreases (a loop from step S25 and step S26 to step S24).

As a result of increasing, stepwise, the electric energy EQ (the duty ratio) supplied to the electric fan (step S18 and step S21), when actual rotating speed is smaller than the set rotating speeds R2 and R3 by a predetermined amount or more and an actual current value is larger than the set current values A2 and A3 by a predetermined amount or more ("y2" in step S20 or in step S23), it is assumed that the clogging decreases. The electric energy EQ (the duty ratio) supplied to the electric fan is reduced stepwise (step S18 or step S15).

When the actual rotating speed is smaller than the initial rotating speed R1 by a predetermined amount or more and the actual current value is larger than the initial current value A1 by a predetermined amount or more ("y2" in step S17), assuming that clogging has occurred, the second control section 90 controls the management-side output section 80 to output the replacement attention information I1, which is the alarm about replacement of the air filter 21, (step S24) and continues the operation at a slightly larger current value while subsequently keeping a state in which the replacement attention information I1 is output unless the clogging decreases (the loop from step S25 and step S26 to step S24).

Note that, since a decrease in the clogging is considered to actually less easily occur, when the replacement attention information I1 is output in step S24, it is also possible to stop the fan control processing and only continue to emit the alarm.

Referring back to the main processing shown in FIG. 9, in step S7, predetermined output control processing is executed. In the output control processing in step S7, the second control section 90 controls the management-side output section 80 to output various kinds of information including the replacement attention information I1, the action disability warning information 12, the charging attention information 13, and the heatstroke warning information 14. In step S8, it is determined whether a predetermined time (for example, 2 seconds) has elapsed. When the predetermined time has elapsed, the protective system for respiration changes to a sleep state (step S9) and the processing is ended.

As explained above with reference to FIGS. 1 to 10, with the protective equipment for respiration in the embodiment, the detecting section 30 detects a physical quantity concerning at least one of a work environment of an operator, an action state of the operator, and an operation state of the electric fan 22, the first control section 40 controls the electric fan 22 based on a detection result of the detecting section 30, and the first communication section 50 transmits first information indicating the detection result of the detecting section 30 or second information concerning an operation amount on a control target of the first control section 40 to the management terminal 2 functioning as the management device, which is the external device in the claims. Therefore, it is possible to assist equipment management for stably supplying filtered air to the operator or safety management for securing safety of work performed while receiving the supply of the filtered air such that the equipment management or the safety management can be appropriately performed even from a remote place.

As explained with reference to FIGS. 1 to 10, with the protective equipment for respiration in the embodiment, the detecting section 30 includes the air volume sensor 31 that detects the supplied air volume SV of the filtered air, the first control section 40 adjusts, based on a detection result of the air volume sensor 31, the electric energy EQ supplied to the electric fan 22 such that the supplied air volume SV reaches the predetermined reference air volume RV, and the first communication section 50 transmits the second information indicating the adjustment amount DA of the electric energy EQ adjusted by the first control section 40 to the management terminal 2 as the replacement period related information RI1. The replacement period related information RI1 is information related to a replacement period of an air filter.

Therefore, with the protective equipment for respiration in the embodiment, in the external device placed in a remote place, it is possible to learn a degree of clogging of the air filter 21 based on the adjustment amount DA of the electric energy EQ for setting the supplied air volume SV to the reference air volume RV. It is possible to assist the equipment management for stably supplying the filtered air to the operator such that the equipment management can be more appropriately performed even from a remote place.

For example, the electric energy EQ with which the supplied air volume SV reaches the reference air volume RV when the air filter 21 is not clogged is represented as electric energy EQ0 and the electric energy EQ with which the supplied air volume SV actually reaches the reference air volume RV is represented as electric energy EQ1. At this time, the adjustment amount DA of the electric energy EQ is DA: (EQ1-EQ0). If the adjustment amount DA is equal to or larger than the predetermined threshold TS for determining clogging of the air filter 21, in the external device placed in the remote place, it is possible to learn that clogging occurs in the air filter 21 and the replacement period has come. It is possible to assist the equipment management for stably supplying the filtered air to the operator such that the equipment management can be more appropriately performed even from a remote place.

As explained with reference to FIGS. 1 to 10, with the protective equipment for respiration in the embodiment, the detecting section 30 includes the voltage sensor 32, the voltage sensor 32 detects the terminal voltage TV of the first battery 26a and the second battery 26b, and the first communication section 50 transmits the first information indicating the terminal voltage TV detected by the voltage sensor 32 to the management terminal 2 as the residual power related information RI2. The residual power related information RI2 is information related to residual power of the first battery 26a and the second battery 26b. Therefore, with the protective equipment for respiration in the embodiment, it is possible to check, in the external device placed in the remote place, the residual power of the first battery 26a and the second battery 26b. It is possible to assist the equipment management for stably supplying the filtered air to the operator such that the equipment management can be more appropriately performed even from a remote place.

As explained with reference to FIGS. 1 to 10, with the protective equipment for respiration in the embodiment, the detecting section 30 includes the acceleration sensor 33, the acceleration sensor 33 detects the acceleration AR about the fan unit 20, and the first communication section 50 transmits the first information indicating the acceleration AR to the management terminal 2 functioning as the external device as the action disability determination information RI3. The action disability determination information RI3 is information for determining whether the operator has fallen down and has become disabled to act. Therefore, with the protective equipment for respiration in the embodiment, in the external device placed in the remote place, it is possible to check whether the operator has fallen down and has become disabled to act. It is possible to take quick measures in a crisis of the operator. It is possible to assist safety management for securing safety of work performed while receiving the supply of the filtered air such that the safety management can be more appropriately performed even from a remote place.

As explained with reference to FIGS. 1 to 10, with the protective equipment for respiration in the embodiment, the detecting section 30 includes the temperature sensor 34, the temperature sensor 34 detects the air temperature TP in the work environment of the operator and the first communication section 50 transmits the first information indicating the air temperature TP detected by the temperature sensor 34 to the management terminal 2 functioning as the external device as the heat stress index related information RI4. The heat stress index related information RI4 is information related to calculation of a heat stress index. Therefore, with the protective equipment for respiration in the embodiment, in the external device placed in the remote place, it is possible to calculate a heat stress index in a work site. It is possible to work out, based on the calculated heat stress index, a work schedule such as desired lengths of work times and rest intervals of operators set in advance. It is possible to assist safety management for securing safety of work performed while receiving the supply of the filtered air such that the safety management can be more appropriately performed even from a remote place.

As explained with reference to FIGS. 1 to 10, with the protective system for respiration and the management method in the embodiment, the second communication section 70 of the management terminal 2 functioning as the management device receives the first information indicating the detection result of the detecting section 30 or the second information concerning the operation amount on the control target of the first control section 40 transmitted by the first communication section 50, the management-side output section 80 outputs, as visual information or auditory information, third information concerning at least one of a work environment of the operator, an action state of the operator, and an operation state of the electric fan 22, and the second control section 90 controls the management-side output section 80 to output the third information based on the first information or the second information received by the second communication section 70. Therefore, with the protective system for respiration and the management method in the embodiment, in the management terminal 2 placed in a remote place, it is possible to assist equipment management for stably supplying filtered air to the operator or safety management for securing safety of work performed while receiving the supply of the filtered air such that the equipment management or the safety management can be appropriately performed even from a remote place.

As explained with reference to FIGS. 1 to 10, with the protective system for respiration and the management method in the embodiment, when the adjustment amount DA indicated by the second information is equal to or larger than the threshold TS, the second control section 90 controls the management-side output section 80 to output the replacement attention information I1 related to a replacement period of the air filter as the third information. The threshold TS is a threshold for determining clogging of the air filter 21. Therefore, it is possible to accurately determine based on scientific grounds whether clogging has occurred in the air filter 21 and replace the air filter 21 in an optimum period. It is possible to assist equipment management for stably supplying filtered air to the operator such that the equipment management can be appropriately performed even from a remote place.

As explained with reference to FIGS. 1 to 10, with the protective system for respiration and the management method in the embodiment, when a change in the acceleration AR indicated by the first information matches the predetermined action disability determination pattern JP, the second control section controls the management-side output section 80 to output the action disability warning information 12 as the third information. The action disability determination pattern JP indicates that the operator has fallen down and has become disabled to act. Therefore, it is possible to learn in the management terminal 2 placed in a remote place that the operator has fallen down and has become disabled to act because of heatstroke or the like. It is possible to take quick measures in a crisis of the operator. It is possible to assist safety management for securing safety of work performed while receiving the supply of the filtered air such that the safety management can be more appropriately performed even from a remote place.

The embodiment of the present invention is explained above with reference to the drawings (FIGS. 1 to 10). However, the present invention is not limited to the embodiment explained above. It is possible to carry out the invention in various modes without departing from the gist of the present invention (for example, (1) described below).
(1) In the embodiment, when the adjustment amount DA for the electric energy EQ is equal to or larger than the threshold TS, the second control section 90 controls the management-side output section 80 to output the replacement attention information I1 serving as the third information. However, when the actual supplied air volume SV decreases by a fixed amount or more from the supplied air volume SV assumed at a predetermined electric energy EQ, the second control section 90 may control the management-side output section 80 to output the replacement attention information I1 serving as the third information.

### Reference Signs List

- SV: Supplied air volume
- RV: Reference air volume
- EQ: Electric energy
- DA: Adjustment amount
- JP: Action disability determination pattern
- I1: Replacement attention information
- 12: Action disability warning information
- RI1: Replacement period related information
- RI2: Residual power related information
- RI3: Action disability determination information
- RI4: Heat stress index related information
- 1: Protective equipment for respiration
- 2: Management terminal
- 10: Protective equipment main body
- 20: Fan unit
- 21: Air filter
- 22: Electric fan
- 23: Hose
- 26: Power supply section
- 26a: First battery
- 26b: Second battery
- 30: Detecting section
- 31: Air volume sensor
- 32: Voltage sensor
- 33: Acceleration sensor
- 34: Temperature sensor
- 40: First control section
- 50: First communication section
- 70: Second communication section
- 80: Management-side output section (information output means, information output section)
- 90: Second control section

## Claims

1. Protective equipment for respiration comprising:
a protective equipment main body configured to cover a face of an operator;
a fan unit including an electric fan and an air filter that filters air, the electric fan blowing filtered air, which is the air filtered by the air filter, to the protective equipment main body via ventilation means such as a hose;
a detecting section provided in the fan unit and configured to detect a physical quantity concerning at least one of a work environment of the operator, an action state of the operator, and an operation state of the electric fan;
a first control section provided in the fan unit and configured to control the electric fan based on a detection result of the detecting section; and
a first communication section provided in the fan unit and configured to transmit first information indicating a detection result of the detecting section or second information concerning an operation amount on a control target of the first control section to an external device including information output means.

2. The protective equipment for respiration according to claim 1, wherein
the detecting section includes an air volume sensor that detects a supplied air volume of the filtered air blown to the protective equipment main body via the ventilation means,
the first control section adjusts, based on a detection result of the air volume sensor, electric energy supplied to the electric fan such that the supplied air volume of the filtered air reaches a predetermined reference air volume, and
the first communication section transmits the second information indicating an adjustment amount of the electric energy adjusted by the first control section to the external device as replacement period related information related to a replacement period of the air filter.

3. The protective equipment for respiration according to claim 1 or 2, further comprising a power supply section including at least one battery and configured to supply electric power to the electric fan, wherein
the detecting section includes a voltage sensor that detects a terminal voltage of the at least one battery, and
the first communication section transmits the first information indicating the terminal voltage detected by the voltage sensor to the external device as residual power related information related to residual power of the at least one battery.

4. The protective equipment for respiration according to any one of claims 1 to 3, wherein
the detecting section includes an acceleration sensor that detects acceleration about the fan unit involved in movement of the operator, and
the first communication section transmits the first information indicating the acceleration detected by the acceleration sensor to the external device as action disability determination information for determining whether the operator has fallen down and has become disabled to act.

5. The protective equipment for respiration according to any one of claims 1 to 4, wherein
the detecting section includes a temperature sensor that detects an air temperature in the work environment of the operator, and
the first communication section transmits the first information indicating the air temperature detected by the temperature sensor to the external device as heat stress index related information related to calculation of a heat stress index.

6. A protective system for respiration comprising:
the protective equipment for respiration according to any one of claims 1 to 5; and
a management device functioning as the external device, the management device including:
a second communication section configured to receive the first information or the second information transmitted by the first communication section;
an information output section functioning as the information output means and configured to output third information concerning at least one of a work environment of the operator, an action state of the operator, and an operation state of the electric fan as visual information or auditory information; and
a second control section configured to control, based on the first information or the second information received by the second communication section, the information output section to output the third information.

7. The protective system for respiration according to claim 6, wherein
the detecting section includes an air volume sensor that detects a supplied air volume of the filtered air blown to the protective equipment main body via the ventilation means,
the first control section adjusts, based on a detection result of the air volume sensor, electric energy supplied to the electric fan such that the supplied air volume of the filtered air reaches a predetermined reference air volume,
the first communication section transmits the second information indicating an adjustment amount of the electric energy adjusted by the first control section to the management device as replacement period related information related to a replacement period of the air filter,
the second communication section receives the second information indicating the adjustment amount of the electric energy as the replacement period related information, and
when the adjustment amount indicated by the second information is equal to or larger than a predetermined threshold for determining clogging of the air filter, the second control section controls the information output section to output replacement attention information for urging replacement of the air filter as the third information.

8. The protective system for respiration according to claim 6 or 7, wherein
the detecting section includes an acceleration sensor that detects acceleration about the fan unit,
the first communication section transmits the first information indicating the acceleration detected by the acceleration sensor to the management device as action disability determination information for determining whether the operator has fallen down and has become disabled to act,
the second communication section receives the first information indicating the acceleration as the action disability determination information for determining whether the operator has fallen down and has become disabled to act, and
when a change in the acceleration indicated by the first information matches a predetermined action disability determination pattern indicating that the operator has fallen down and has become disabled to act, the second control section controls the information output section to output action disability warning information warning that the operator has fallen down and has become disabled to act as the third information.

9. A management method for, using the protective equipment for respiration according to any one of claims 1 to 5, performing equipment management for stably supplying filtered air to an operator or safety management for securing safety of work performed while receiving the supply of the filtered air, the management method comprising:
a first step of receiving the first information or the second information transmitted by the first communication section;
a second step of the information output means outputting third information concerning at least one of a work environment of the operator, an action state of the operator, and an operation state of the electric fan as visual information or auditory information; and
a third step of controlling, based on the first information or the second information received in the first step, the information output means to output the third information.
